# EUROPEAN PATENT APPLICATION

(11) **EP 4 837 535 A1**
(43) Date of publication of application: **11.11.2026**
(21) Application number: 25174189.8
(22) Date of filing: 05.05.2025
(51) Int. Cl.: A61B 90/00, A61B 34/00

(54) **SURGICAL DATA FUSION SYSTEM, SURGICAL IMAGING SYSTEM, AND CORRESPONDING METHOD AND COMPUTER PROGRAM**

(71) Applicant: Leica Instruments (Singapore) Pte Ltd., Singapore 618299 (SG)
(72) Inventor: THEMELIS, George, 618299 Singapore (SG)
(74) Representative: 2SPL Patentanwälte PartG mbB

(57) **Abstract**

Examples relate to a surgical data fusion system, to a surgical imaging system comprising a surgical data fusion system, a surgical data fusion method and a corresponding computer program. The surgical data fusion system is configured to obtain three-dimensional anatomical information of a patient, the three-dimensional anatomical information being based on a pre-operative or intraoperative scan of a patient, obtain one or more intraoperative measurements of the patient, and determine position information for a visual representation (40) of the one or more intraoperative measurements relative to the three-dimensional anatomical information, wherein the position information indicates a position of the visual representation (40) of the one or more intraoperative measurements relative to at least one planar visual representation (30) of at least one slice of the three-dimensional anatomical information.

## Description

### Technical field

Examples relate to a surgical data fusion system, to a surgical imaging system comprising a surgical data fusion system, a surgical data fusion method, and a corresponding computer program.

### Background

Surgeons, particularly neurosurgeons and radiologists, are trained to interpret and understand three-dimensional (3D) anatomical data through radiological cross-sectional images in axial, coronal, and sagittal planes. These views allow for precise spatial comprehension of anatomical and pathological structures.

Current Image-Guided Surgery (IGS) systems in surgical microscopes primarily overlay preoperative 3D data onto live optical images from the microscope, presenting a hybrid optical-3D visualization based on the optical perspective. While this provides realistic views of the surgical field, it may be less effective in conveying precise 3D spatial relationships between structures relative to the field of view.

Several methods have been explored to enhance surgical visualization, but each may have limitations. One approach involves optical perspective overlays. In this approach, preoperative 3D imaging data is overlaid onto the live optical feed from the surgical microscope, aligning it with the microscope's optical perspective. However, the optical perspective may not readily convey the depth and spatial relationships as effectively as radiological cross-sections do. Furthermore, surgeons may find interpreting the overlaid images challenging, because they differ from familiar radiological visualization formats. Finally, the lack of precise spatial cues can reduce precision when measuring distances and understanding the location of critical structures.

Another approach utilizes separate displays for radiological images. This involves viewing preoperative radiological images, such as axial, coronal, and sagittal planes, on separate monitors during surgery. However, this method creates a disjointed workflow, as surgeons must shift their attention between the surgical field and the monitors, interrupting their concentration. It also requires surgeons to mentally map the 2D radiological images to the 3D surgical field, increasing cognitive load. Finally, it may be difficult to share precise spatial information with the surgical team in real-time using this method.

While some approaches have addressed creating 3D surface maps of the surgical cavity using stereoscopic imaging, they do not focus on integrating radiological cross-sectional visualization with live optical imaging in a manner that mimics the familiar radiology formats.

There may be a desire to provide an improved concept for the visualization of pre-operative 3D data during surgery.

### Summary

This desire is addressed by the subject matter of the independent claims.

Various examples of the present disclosure are based on the finding, that, in many IGS systems, surgeons may lack an intuitive and precise method to visualize and measure 3D quantitative spatial relationships during surgery in a format familiar from radiology. While the visualization often employed is based on projecting a visualization of the 3D data onto the live optical images, to integrate them with the live optical view, this approach does not provide as good spatial comprehension of anatomical and pathological structures as the views through radiological cross-sectional images in axial, coronal, and sagittal planes. In the proposed concept, a joint visualization of a 3D pre- or intraoperative scan with intraoperative measurements is prepared or provided, in which a visualization of the intraoperative measurements is placed relative to at least one planar visual representation of the 3D pre-or intraoperative scan, e.g., as known from radiological cross-sectional images in axial, coronal, and sagittal planes. This way, an improved visualization can be provided that improves the surgeon's understanding of the 3D anatomical data.

Some aspects of the present disclosure relate to a surgical data fusion system. The surgical data fusion system comprises one or more processors and one or more storage devices. The surgical data fusion system is configured to obtain three-dimensional anatomical information of a patient. The three-dimensional anatomical information is based on a pre-operative or intraoperative scan of a patient. The surgical data fusion system is configured to obtain one or more intraoperative measurements of the patient. The surgical data fusion system is configured to determine position information for a visual representation of the one or more intraoperative measurements relative to the three-dimensional anatomical information. The position information indicates a position of the visual representation of the one or more intraoperative measurements relative to at least one planar visual representation of at least one slice of the three-dimensional anatomical information. This way, an improved visualization can be provided that improves the surgeon's understanding of the 3D anatomical data.

The proposed concept is based on mimicking the views provided by radiological cross-sectional images, which are familiar to surgeons. In other words, the at least one planar visual representation may mimic at least one cross-sectional radiological image. For example, the at least one planar visual representation may represent at least one of an axial plane, a coronal plane, or a sagittal plane of the three-dimensional anatomical information. This way, the surgeon is provided with a view that he or she is trained to understand.

In general, the actual surgical visualization can be provided by another system or platform. In this case, the surgical data fusion system may be configured to provide the position information to the system or platform providing the surgical visualization. Alternatively, the surgical data fusion system may be a surgical visualization system that is configured to provide the surgical visualization. In other words, the surgical data fusion system (or surgical visualization system) may be configured to generate a surgical visualization of a surgical site. The surgical visualization may comprise the at least one planar visual representation of the at least one slice of the three-dimensional anatomical information, overlaid with the visual representation of the one or more intraoperative measurements. The overlay may be placed on the at least one planar visual representation based on the position information. The surgical data fusion system may be configured to provide the surgical visualization for a display device. This way, an improved visualization is provided to the surgeon that improves the surgeon's understanding of the 3D anatomical data.

Radiological cross-sectional images each represent a slice through a 3D representation of the anatomy of the patient, according to one of three pre-defined axes. If the respective radiological cross-sectional images of the axial plane, coronal plane, or sagittal plane are stacked on top or next to each other, they represent the 3D representation of the anatomy, minus the portions that are located between the slices. To get to a portion of the anatomy that is of interest to the surgeon, the user (surgeon) may be provided with a mechanism to select one or more of the slices, effectively "scrolling" through the stack of slices to arrive at a slice of interest. In other words, the surgical data fusion system may be configured to obtain a user input of (e.g., from) a user of the surgical data fusion system, and to select the at least one slice of the three-dimensional anatomical information for the surgical visualization based on the user input. This way, at least one slice of interest may be selected for the visualization, enabling the surgeon to tune the visualization to the needs of the surgery at hand.

There are various types of intraoperative measurements that can be used to generate a combined surgical visualization together with the slice(s) of the three-dimensional anatomical information. For example, the one or more intraoperative measurements of the patient may comprise image data of (e.g., from) a surgical imaging device. For example, the image data may show the surgical site. The visual representation of the one or more intraoperative measurements may be based on the image data. In this way, the live image data of the surgical imaging device (e.g., a surgical microscope) can be overlaid over the slice(s) of the three-dimensional anatomical information, providing the surgeon with a valuable mechanism for planning the next steps during the surgical procedure.

In general, the visualization of the live view on the surgical site can be improved by ensuring that the surgeon can spatially interpret the surgical site. For this purpose, an intermediate representation of the surgical site may be created that is based on a three-dimensional depth map of the surgical site. For example, the surgical data fusion system may be configured to use image projection to map the image data onto a three-dimensional depth map of the surgical site. The visual representation of the one or more intraoperative measurements may be based on the image data mapped onto the three-dimensional depth map of the surgical site. By providing a visual representation that is based on the three-dimensional depth map of the surgical site, the spatial understanding of the surgeon may be improved. Moreover, image data from multiple angles of view may be combined to provide a more comprehensive view of the surgical site.

In more general terms, the surgical data fusion system may be configured to determine a three-dimensional depth map of the surgical site and generate the surgical visualization based on the three-dimensional depth map of the surgical site. By providing a surgical visualization that is based on the three-dimensional depth map of the surgical site, the spatial understanding of the surgeon may be improved.

Fusing the planar visual representation of the at least one slice and the visual representation of the one or more intraoperative measurements being represented by a three-dimensional depth map might not be straightforward, as it involves placing a 3D object relative to a plane, with the plane overlapping with the 3D object at the surgical site. For example, to perform such a combined 3D-on-2D visualization, the shape of the surgical site (as represented by the three-dimensional depth map) at the position (e.g., depth within the stack) of the respective slice may be considered. In other words, the surgical data fusion system may be configured to generate the visual representation of the one or more intraoperative measurements based on the shape of the surgical site at the position of the at least one slice of the three-dimensional anatomical information. In particular, a portion of the respective slice(s) may be removed to make space for the visual representation of the surgical site, as represented by the three-dimensional depth map. For example, the surgical site may appear to extend above and/or below the plane(s) defined by the respective slice(s), with the slice(s) having a cut-out that represents the current shape of the surgical site, as represented by the three-dimensional depth map. This way, the 3D representation of the one or more intraoperative measurements can be integrated with the planar view on the three-dimensional anatomical information.

In many surgical procedures, tissue or bones are permanently or temporarily removed from the patient, for example, as a tumor is removed or to make space during the surgical procedure. These removals may be reflected in the surgical visualization. For example, the surgical data fusion system may be configured to determine, based on the three-dimensional anatomical information and the three-dimensional depth map of the surgical site, a portion of the surgical site that is removed compared to the pre-operative scan. The surgical data fusion system may be configured to generate the at least one planar visual representation of the at least one slice of the three-dimensional anatomical information without the portion of the surgical site that is removed. This way, the surgical visualization more accurately represents the current progress of the surgical procedure, providing more valuable real-time information and context for the surgeon.

Some differences between the three-dimensional anatomical information and the current position of the tissue may occur without being caused by the surgery. For example, during neurosurgical procedures, brain shift occurs. Brain shift refers to the movement or deformation of brain tissue that occurs during a neurosurgical procedure. This can happen due to several factors, such as changes in cerebrospinal fluid pressure, gravity, and manipulation of brain tissues during surgery. Brain shift poses challenges for surgeons because it can alter the position of anatomical landmarks, potentially affecting the accuracy of the three-dimensional anatomical information. In the proposed concept, the three-dimensional depth map may be used to mitigate brain shift and similar phenomena. In other words, the surgical data fusion system may be configured to determine, based on the three-dimensional anatomical information and the three-dimensional depth map of the surgical site, a shift or deformation of at least a portion of the surgical site. The surgical data fusion system may be configured to adjust the at least one planar visual representation of the at least one slice of the three-dimensional anatomical information based on the shift or deformation of least the portion of the surgical site. This way, the three-dimensional anatomical information is adjusted to the current state of the anatomy of the patient, resulting in a more precise visualization.

There are various approaches for determining the three-dimensional depth map. For example, the surgical data fusion system may use sensor data of (e.g., from) a depth sensor, such as a time-of-flight sensor or a structured light-based sensor. In other words, the surgical data fusion system may be configured to determine the three-dimensional depth map using at least one of sensor data of (e.g., from) a time-of-flight sensor or image data (e.g., of/from the surgical imaging device or another imaging device) showing a structured light projection. This is one way to determine the three-dimensional depth map.

Alternatively, or additionally, the surgical data fusion system may be configured to determine the three-dimensional depth map of the surgical site using stereo disparity analysis based on image frames contained in the image data of (e.g., from) the surgical imaging device showing the surgical site from different angles of view. For example, the stereo disparity analysis may be augmented by projecting structured light onto the surgical site and using information from the structured light projection for determining the three-dimensional depth map. This way, the three-dimensional depth map can be created without requiring additional sensors.

Stereo disparity analysis refers to the process of determining the difference in images taken from two slightly different viewing angles, akin to human binocular vision, to compute depth information about a scene. These viewing angles may be present in stereoscopic image data, which is often generated by surgical microscopes. Accordingly, the image data may comprise stereoscopic image data. The surgical data fusion system may be configured to determine the three-dimensional depth map of the surgical site using stereo disparity analysis based on the stereo image frames contained in the stereoscopic image data. This way, the stereoscopic image data generated by existing imaging modalities may be used for determining the three-dimensional depth map, in real-time, without requiring the surgical imaging device to sequentially take on different viewing angles.

If stereoscopic image data is not available, or to further augment the precision of the three-dimensional depth map, the viewing angle of the surgical imaging device may be changed to capture the surgical site from different viewing angles. For example, the surgical data fusion system may be configured to control a surgical imaging system to change the angle of view of a surgical imaging device to obtain the image frames showing the surgical site from different angles of view. This is another approach for generating image data that is suitable for stereo disparity analysis.

In some examples, the surgical data fusion system may retrieve the 3D three-dimensional anatomical information of (e.g., from) an IGS system. During surgery, IGS systems often track the position and configuration of the surgical site in order to provide appropriate output. This process is called registration. Registration is the process of aligning preoperative images (such as CT, MRI, or ultrasound scans) with the patient's actual anatomy during surgery. This functionality can be leveraged by the surgical data fusion system for determining the position information. For example, the surgical data fusion system may be configured to obtain the three-dimensional anatomical information of (e.g., from) an image-guided surgery system. The surgical data fusion system may be configured to determine the position information based on the registration functionality of the image-guided surgery system. This way, the capabilities of an existing system can be reused for performing the surgical data fusion.

During surgery, a major aspect is the concurrent visualization of the three-dimensional anatomical information and the live images of (e.g., from) the surgical imaging device. However, there are other measurements that can also be of interest during surgery. For example, the one or more intraoperative measurements of the patient may comprise functional imaging data representing brain activity of the patient. For example, the three-dimensional anatomical information may comprise three-dimensional anatomical information of the patient's brain. By visualizing the brain activity of the patient with reference to the three-dimensional anatomical information, the surgeon can be provided with useful information during surgery.

Another aspect of the present disclosure relates to a surgical imaging system comprising a surgical imaging device and the surgical data fusion system. For example, the surgical data fusion system may be used to provide or assist with the surgical visualization during surgical procedures. This way, an improved visualization can be provided that improves the surgeon's understanding of the 3D anatomical data.

The proposed concept is particularly useful in neurosurgery, as neurosurgeons are used to interpret radiological cross-sectional images of the brain. Accordingly, the surgical imaging system may be a neurosurgical imaging system. The three-dimensional anatomical information may comprise three-dimensional anatomical information of the brain of the patient. The one or more intraoperative measurements may relate to the brain of the patient.

Some aspects of the present disclosure relate to a surgical data fusion method. The method comprises obtaining three-dimensional anatomical information of a patient. The three-dimensional anatomical information is based on a pre-operative or intraoperative scan of a patient. The method comprises obtaining one or more intraoperative measurements of the patient. The method comprises determining position information for a visual representation of the one or more intraoperative measurements relative to the three-dimensional anatomical information. The position information indicates a position of the visual representation of the one or more intraoperative measurements relative to at least one planar visual representation of at least one slice of the three-dimensional anatomical information.

Another aspect of the present disclosure relates to a computer program having a program code for performing the method when the program is executed on a processor.

### Short description of the Figures

Some examples of apparatuses and/or methods will be described in the following by way of example only, and with reference to the accompanying figures, in which:
- Fig. 1a: shows a block diagram of an example of a surgical data fusion system or surgical visualization system;
- Fig. 1b: shows a schematic diagram of a surgical imaging system comprising a surgical data fusion system;
- Fig. 1c: shows an illustration of a visualization comprising a planar visual representation of a slice of three-dimensional anatomical information, overlaid with a visualization of live images of a surgical microscope;
- Fig. 2: shows an example of radiological cross-sections augmented with real-time optical imaging;
- Fig. 3a: illustrates the planes defining radiological cross-sectional views;
- Fig. 3b: shows a combined view comprising radiological cross-sectional views and a live optical feed;
- Fig. 4: shows a flow chart of an example of a surgical data fusion method;
- Fig. 5: shows a diagram of an example of a system comprising a surgical imaging device and a computer system.

### Detailed Description

Various examples will now be described more fully with reference to the accompanying drawings in which some examples are illustrated. In the figures, the thicknesses of lines, layers and/or regions may be exaggerated for clarity.

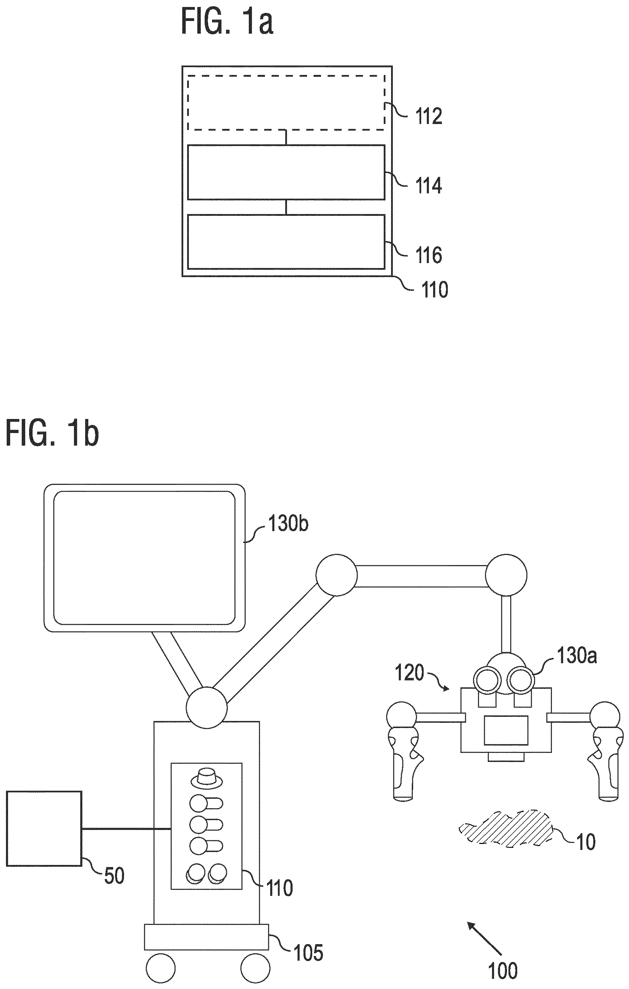

Fig. 1a shows a schematic diagram of an example of a surgical data fusion system 110 (also denoted surgical visualization system or control system), which can be used for a surgical imaging system 100 (shown in Fig. 1b). For example, the surgical data fusion system 110 may be a component of the surgical imaging system 100, or it may be separate from the surgical imaging system 100, e.g., part of an Image-Guided Surgery (IGS) system 50 (shown in Fig. 1b). If the surgical data fusion system is part of the surgical imaging system 100, it may be implemented by an overall computer-based control system of the surgical imaging system and used to control various aspects of the surgical imaging system 100. In particular, it may be used for acquisition and/or processing of imaging data by at least one optical imaging sensor of an imaging device 120 of the surgical imaging system, through various means that will be introduced in more detail in the following. Additionally, the surgical data fusion system 110 may be configured to control additional aspects of the surgical imaging system 100, e.g., to provide a display signal for one or more display devices 130a, 130b of the surgical imaging system, and/or to control and/or execute automatic operations of the surgical imaging system, e.g., using an automated imaging device positioning system such as a robotic arm.

In general, the surgical data fusion system 110 may be a computer system. The surgical data fusion system 110 comprises one or more processors 114 and one or more storage devices 116. Optionally, the surgical data fusion system 110 further comprises one or more interfaces 112. The one or more processors 114 are coupled to the one or more storage devices 116 and to the one or more interfaces 112. In general, the functionality of the surgical data fusion system 110 may be provided by the one or more processors 114, in conjunction with the one or more interfaces 112 (for exchanging data/information with one or more other components of the surgical imaging system 100 and outside the surgical imaging system 100, such as an optical imaging sensor of the imaging device 120 or an IGS system 50), and with the one or more storage devices 116 (for storing information, such as machine-readable instructions of a computer program being executed by the one or more processors). In general, the functionality of the one or more processors 114 may be implemented by the one or more processors 114 executing machine-readable instructions. Accordingly, any feature ascribed to the one or more processors 114 may be defined by one or more instructions of a plurality of machine-readable instructions. The surgical data fusion system 110 may comprise the machine-readable instructions, e.g., within the one or more storage devices 116.

As outlined above, the surgical data fusion system 110 may be part of the surgical imaging system 100, which comprises various components in addition to the surgical data fusion system 110. For example, the surgical imaging system 100 comprises the imaging device 120, and may comprise one or more additional components, such as one or more display devices 130a-130b. Fig. 1b shows a schematic diagram of an example of such a surgical imaging system 100, and in particular of a surgical microscope system 100. In the following, the surgical imaging system 100 may also be referred to as surgical microscope system 100. A surgical microscope system is a surgical imaging system 100 that comprises a (surgical) microscope as the imaging device 120. However, the proposed concept is not limited to such embodiments. The surgical imaging system 100 may be based on various (single or multiple) imaging devices, such as one or more microscopes, one or more endoscopes, and/or one or more exoscopes (also sometimes called extracorporeal telescopes). Exoscopes are camera-based imaging systems, and in particular camera-based 3D imaging systems, which are suitable for providing images of surgical sites with high magnification and a large depth of field. Compared to microscopes, which may be used via oculars, exoscopes are only used via display modalities, such as a monitor or a head-mounted display. Accordingly, the surgical imaging system 100 may alternatively be a surgical endoscope system, or a surgical exoscope system. The following illustrations assume that the surgical imaging device 120 is a surgical microscope and that the surgical imaging system 100 is a surgical microscope system 100.

Accordingly, the surgical imaging system or surgical microscope system 100 may comprise an imaging device, such as a microscope 120. In general, a microscope, such as the microscope 120, is an optical instrument that is suitable for examining objects that are too small to be examined by the human eye (alone). For example, a microscope may provide optical magnification of a sample. In the present concept, the optical magnification is (also) provided for at least one optical imaging sensor. The microscope 120 thus comprises an optical imaging sensor, which is coupled with the system 110. The microscope 120 may further comprise one or more optical magnification components that are used to magnify a view on (e.g., of) the sample, such as an objective (i.e., lens). For example, the surgical imaging device or microscope 120 is often referred to as the 'optics carrier' of the imaging system.

There are a variety of different types of surgical imaging devices. If the imaging device is used in the medical or biological fields, the object being viewed through the imaging device may be a sample of organic tissue, e.g., arranged within a petri dish or present in part of the body of a patient. In various examples presented here, the imaging device 120 may be a microscope of a surgical microscope system, i.e., a microscope that is to be used during a surgical procedure, such as a neurosurgical procedure (i.e., brain surgery). Accordingly, the sample being viewed through the surgical imaging device may be a sample of organic tissue of a patient and may in particular be the surgical site that the surgeon operates on during the surgical procedure, e.g., the brain. However, the proposed concept is also suitable for other types of surgery, such as eye surgery or cardiac surgery.

Fig. 1b shows a schematic diagram of an example of a surgical imaging system 100, and in particular a surgical microscope system 100, comprising the surgical data fusion system 110 and a microscope 120. The surgical microscope system 100 shown in Fig. 1b comprises a number of optional components, such as a base unit 105 (comprising the surgical data fusion system 110) with a (rolling) stand, an auxiliary display 130b that may be arranged at the base unit, ocular displays 130a that are arranged at the surgical microscope 120, and an arm that holds the microscope 120 in place, and is coupled to both the base unit and the microscope 120. In general, these optional and non-optional components may be coupled to the surgical data fusion system 110 which may be configured to control and/or interact with the respective components.

In the proposed concept, the surgical data fusion system 110 is used for aligning slices of three-dimensional pre-operative or intraoperative anatomical data (similar to cross-sections of radiological images) with intraoperative measurements to visualize them in a combined view. This visualization does not necessarily have to be performed by the surgical data fusion system 110. Instead, in some examples, an existing separate platform may be used.

The proposed concept starts with two sets of data to be visualized together: the pre- or intraoperative 3D anatomical data, and the intraoperative measurements. Accordingly, the surgical data fusion system 110 is configured to obtain three-dimensional anatomical information of a patient (based on a pre-operative or intraoperative scan of the patient) and one or more intraoperative measurements of the patient.

For example, the three-dimensional anatomical information may be based on a pre-operative or intraoperative MRI (Magnetic Resonance Imaging) scan, CT (Computed Tomography) scan, or other 3D scan of the anatomy of the patient. For example, the three-dimensional anatomical information may not be limited to the surface of the surgical site and may also include a 3D representation of sub-surface anatomical structures. For example, the three-dimensional anatomical information may include a three-dimensional model of the anatomy of the patient (e.g., at the surgical site). In many cases, the surgical data fusion system 110 may be configured to obtain the three-dimensional anatomical information from an Image Guided Surgery (IGS) system 50. IGS is a technology that uses pre-operative and intraoperative imaging to provide real-time anatomical guidance during surgical procedures. An IGS system typically utilizes (3D) data from modalities such as computed tomography (CT), magnetic resonance imaging (MRI), and sometimes ultrasound, to create a patient-specific anatomical model. This patient-specific anatomical model may be used as three-dimensional anatomical information.

Various types of intraoperative measurement(s) may be visualized together with the three-dimensional anatomical information. For example, in a surgical imaging system, such as the one shown in Fig. 1b, the one or more intraoperative measurements may comprise (live, i.e., periodically updated, e.g., at a frame rate of more than 1 frame per second) image data of (e.g., from) a surgical imaging device, with the image data showing the surgical site.

As the proposed concept is particularly suitable for neurosurgery (where cross-sectional radiological images are widely used), the one or more intraoperative measurements may relate to the brain of the patient. While such measurements can be provided using the surgical imaging device 120 (being a neurosurgical imaging device of a neurosurgical imaging system), functional measurements may also be used in the combined visualization. In other words, the one or more intraoperative measurements of the patient may comprise functional imaging data representing brain activity of the patient and the three-dimensional anatomical information. Examples of functional imaging data include data from fMRI (functional Magnetic Resonance Imaging) which shows brain activity by detecting changes associated with blood flow, and EEG (electroencephalography) which measures electrical activity in the brain using electrodes placed on the scalp. PET (Positron Emission Tomography) scans can also represent brain activity by using a radioactive tracer to show metabolic activity. These data can reveal areas of the brain that are active during specific tasks or at rest. For example, the one or more intraoperative measurements may include an fMRI (functional MRI) scan, including a series of measurements representing blood-oxygen-level dependent (BOLD) contrast, indicating active brain regions during a task. Alternatively, or additionally, the one or more intraoperative measurements may include a Diffusion Tensor Imaging scan, which is a variant of an MRI scan. The DTI scan measures the diffusion of water molecules within the brain to map the white matter tracts. Additionally, or alternatively, the one or more intraoperative measurements may include a Positron Emission Tomography (PET) scan, including measurements representing the distribution of a radiotracer to represent metabolic activity or neurotransmitter binding. Additionally, or alternatively, the one or more intraoperative measurements may include a Magnetoencephalography (MEG) measurement, which represents magnetic fields produced by electrical activity in the brain. To align these measurements with the three-dimensional anatomical information, the three-dimensional anatomical information may comprise three-dimensional anatomical information of the brain of the patient.

The surgical data fusion system 110 is designed to align the three-dimensional anatomical information and the intraoperative measurements. For this purpose, the surgical data fusion system 110 is configured to determine position information for a visual representation 40 of the one or more intraoperative measurements relative to the three-dimensional anatomical information. While alignment between different types of pre- and intraoperative measurements may also be performed in other systems, in the proposed concept, the alignment focuses on a specific type of representation of the three-dimensional anatomical information, which is similar to the radiological cross-sectional views that are familiar to surgeons. In particular, the position information indicates a position of the visual representation 40 of the one or more intraoperative measurements relative to at least one planar visual representation 30 of at least one slice of the three-dimensional anatomical information. In other words, the position information is specific to how the intraoperative measurements are to be placed relative to one or more planar slices of the three-dimensional anatomical information. For example, the position information may represent a spatial relationship between the one or more intraoperative measurements (or their visual representation) to the respective slice(s) of the three-dimensional anatomical information (or their planar visual representation). Focusing on the visualization being performed by the surgical data fusion system 110 or by a downstream visualization system, the position information may represent a spatial relationship between the visual representation of the one or more intraoperative measurements and the planar visual representation of the at least one slice of the three-dimensional anatomical information. Optionally, the position information may further indicate an extent (e.g., in 2D or 3D space) of the respective visual representation(s) of the one or more intraoperative measurements and/or of the at least one slice of the three-dimensional anatomical information.

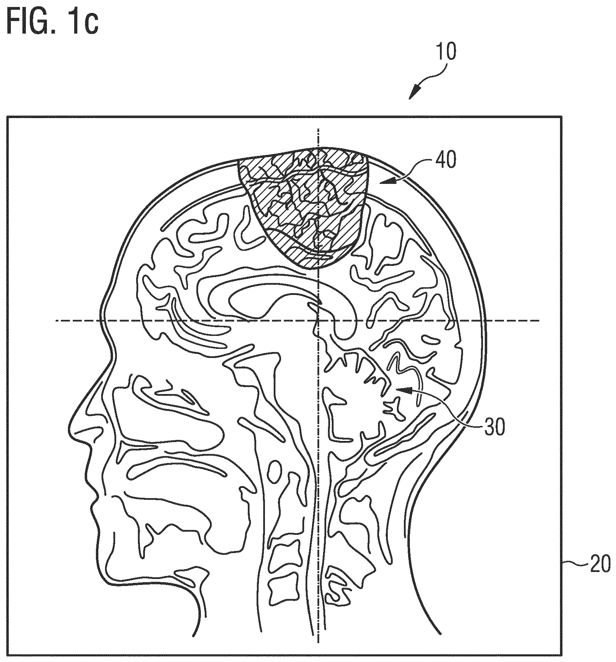

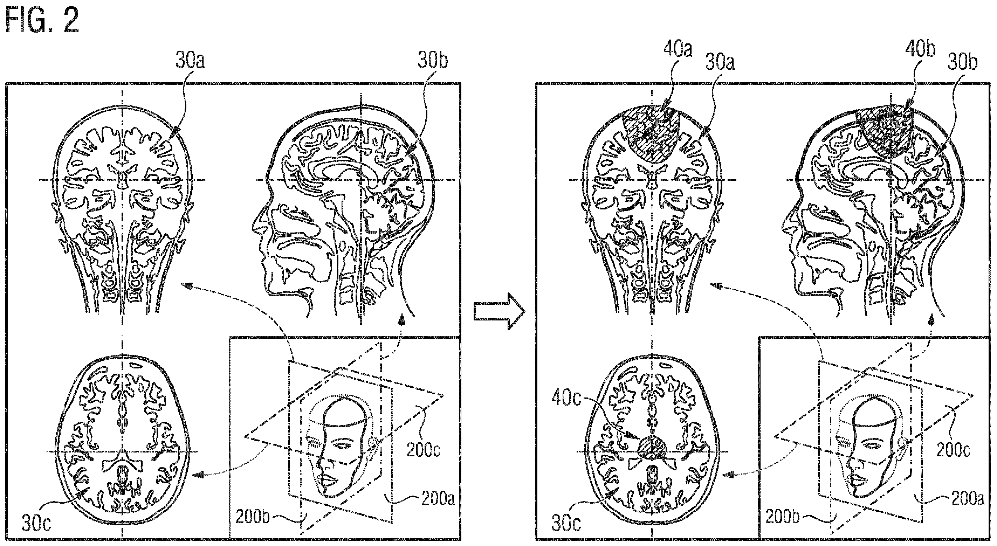

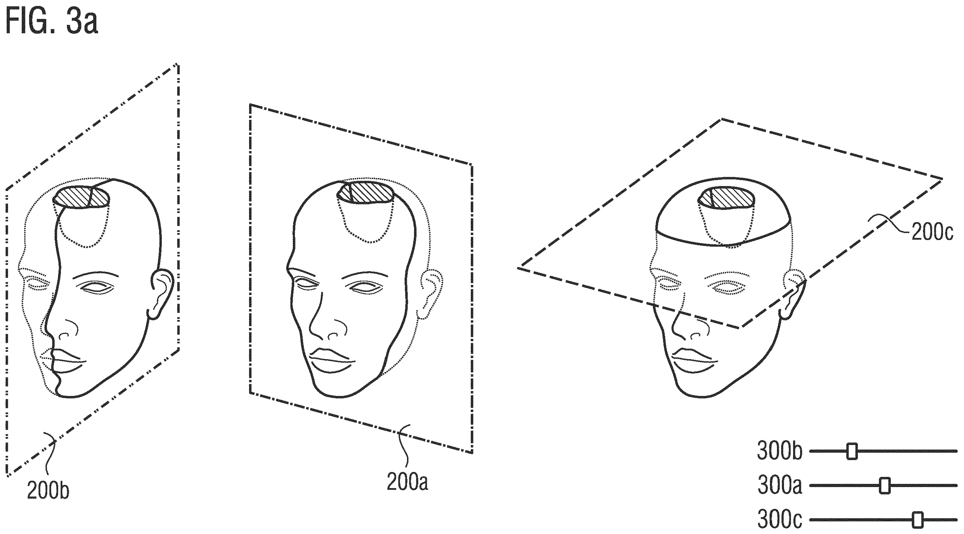

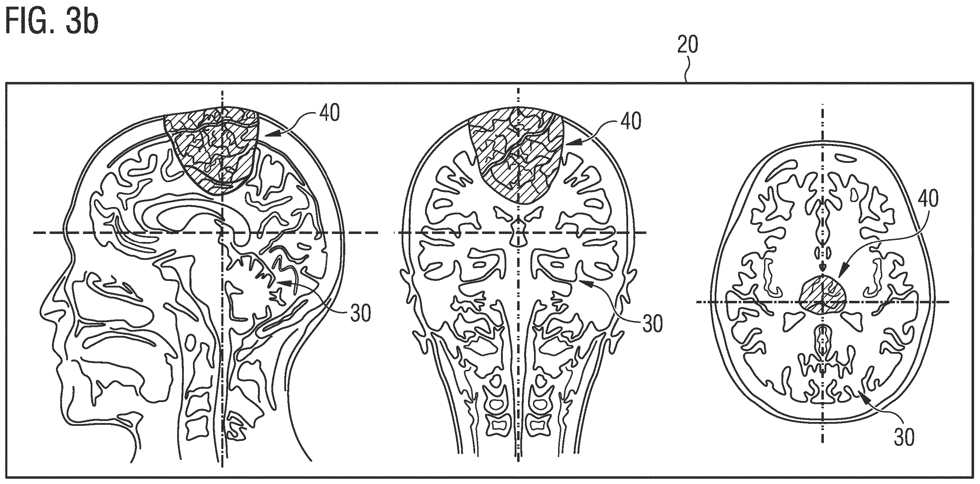

One example of such an alignment and visualization is shown in Fig. 1c (and expanded upon in Figs. 2 and 3a to 3b). Fig. 1c shows an illustration of a visualization 20 comprising a planar visual representation 30 of a slice of the three-dimensional anatomical information, overlaid with a visual representation 40 of live images of a surgical microscope. In Fig. 1c, the slice being shown mimics a cross-sectional view of a sagittal plane of the brain (dividing the brain into left and right sides). At the top of the visualization 20, the live image 40 (i.e., a visual representation of an intraoperative measurement) is overlaid over the slice of the three-dimensional anatomical information (i.e., the cross-sectional view 30 of a sagittal plane of the brain). In the proposed concept, the at least one planar visual representation of the three-dimensional anatomical information mimics at least one cross-sectional radiological image, e.g., as known from the cross-sectional radiological images representing an axial, coronal, or sagittal plane. In other words, the at least one planar visual representation may represent at least one of an axial plane, a coronal plane, or a sagittal plane of the three-dimensional anatomical information. The axial plane (also known as the transverse plane) divides the body into superior and inferior sections top and bottom. The coronal plane (also known as the frontal plane) divides the body into anterior (front) and posterior (back) sections. The sagittal plane divides the body into left and right sections. In Figs. 2 and 3b, examples are given for all three planes. For example, in Fig. 2, planar visual representation 30a represents the coronal plane, planar visual representation 30b represents the sagittal plane, and planar visual representation 30c represents the axial plane. The respective planar visual representations are overlaid with corresponding visual representations of live images of the surgical imaging device 120. For example, multiple planar visual representations (representing different or same types of planes, such as axial, coronal, and sagittal) may be visualized concurrently, or a single planar visual representation may be visualized (and selected by the user, for example).

The planar visual representation(s) of the three-dimensional anatomical information each represents a slice of the three-dimensional anatomical information. In other words, the 3D model of the anatomy of the patient is subdivided into slices, with the planar visual representation representing a cross-section of the anatomy at one of the slices. To obtain a meaningful representation, the user may select the slice or slices being used for the surgical visualization. In other words, the surgical data fusion system may be configured to obtain a user input of a user of the surgical data fusion system and select the at least one slice of the three-dimensional anatomical information for the surgical visualization (and for the determination of the position information) based on the user input. For example, the user may use slider controls to select the at least one slice of the three-dimensional anatomical information, with the slider controls being part of the surgical visualization or provided via haptic user interface elements of the surgical imaging system. Additionally, or alternatively, the surgical visualization may be provided with a user interface element to select which of the coronal plane, sagittal plane, and/or axial plane is to be represented as a planar visual representation in the surgical visualization. Initially, one or more default slices may be automatically selected, e.g., based on the surgical site represented in the one or more intraoperative measurements. For example, the surgical data fusion system 110 may be configured to select one or more slices based on the surgical site represented in the one or more intraoperative measurements, e.g., based on one or more planes aligning with a center point of the surgical site, as represented in the one or more intraoperative measurements.

The determination of the position information is at the core of the proposed concept. As outlined above, the position information indicates a position of the visual representation of the one or more intraoperative measurements relative to at least one planar visual representation of at least one slice of the three-dimensional anatomical information. Thus, the surgical data fusion system 110 may be configured to determine a) the spatial relationship of the one or more intraoperative measurements to the three-dimensional anatomical information as a whole; and b) how and where the one or more intraoperative measurements are to be visualized relative to the at least one planar visual representation of at least one slice of the three-dimensional anatomical information. Operation a) may be performed with the help of the IGS system 50. For example, the surgical data fusion system 110 may be configured to determine the position information (e.g., the spatial relationship of the one or more intraoperative measurements to the three-dimensional anatomical information) based on a registration functionality of the image-guided surgery system.

Alternatively, the surgical data fusion system 110 may be configured to determine the spatial relationship of the one or more intraoperative measurements to the three-dimensional anatomical information using the three-dimensional anatomical information and the one or more intraoperative measurements. For example, the surgical data fusion system 110 may be configured to determine a three-dimensional depth map of the surgical site and determine the spatial relationship between the three-dimensional depth map of the surgical site and the three-dimensional anatomical information (e.g., using anatomical feature matching). For example, the surgical data fusion system 110 may be configured to determine matches between anatomical features included in the three-dimensional depth map and in the three-dimensional anatomical information to determine the spatial relationship. The surgical data fusion system 110 may further be configured to map the one or more intraoperative measurements onto the three-dimensional depth map, and determine the spatial relationship of the one or more intraoperative measurements to the three-dimensional anatomical information based on the spatial relationship between the three-dimensional depth map of the surgical site and the three-dimensional anatomical information and based on the mapping of the one or more intraoperative measurements onto the three-dimensional depth map of the surgical site.

There are various approaches for determining suitable depth maps, such as optical or ultrasound time-of-flight sensors. Accordingly, the surgical data fusion system may be configured to determine the three-dimensional depth map using sensor data of a time-of-flight sensor. As an alternative, the image data of the surgical imaging device may be used to generate the depth map. In other words, the surgical data fusion system 110 may be configured to generate the three-dimensional depth map using stereo disparity analysis based on the image data. In particular, the surgical data fusion system 110 may be configured to determine the three-dimensional depth map of the surgical site using stereo disparity analysis based on image frames contained in image data of a surgical imaging device showing the surgical site from different angles of view. Stereo disparity analysis, also known as stereopsis, is a technique used to determine depth information from two or more images taken from slightly different viewing angles. This process involves identifying corresponding points between the images and calculating the difference in their positions, which is referred to as disparity. The disparity values are then used to estimate the depth map of the surgical site.

In many cases, surgical imaging devices, such as surgical microscopes, are stereoscopic imaging devices, as a stereoscopic view on a surgical site provides improved depth perception for the surgeon. Thus, the image data may comprise stereoscopic image data, showing the surgical site from two slightly different angles of view. The surgical data fusion system 110 may use these different views to generate the three-dimensional depth map using these views. In other words, the surgical data fusion system 110 may be configured to determine the three-dimensional depth map of the surgical site using stereo disparity analysis based on the stereo image frames contained in the stereoscopic image data. Alternatively, or additionally, the surgical imaging device may be moved to obtain different angles of view. In other words, the surgical data fusion system 110 may be configured to control the surgical imaging system 100 to change the angle of view of a surgical imaging device 120 (e.g., using a robotic arm) to obtain the image frames showing the surgical site from different angles of view.

Structured light projection may be used to perform or improve the generation of the three-dimensional depth map. Thus, the surgical data fusion system may be configured to determine the three-dimensional depth map using image data showing a structured light projection. With structured light projection, a three-dimensional depth map can be determined by projecting a known pattern of light - typically lines or a grid - onto the surgical site. Thus, generating a three-dimensional map of sample using structured light may include projecting a series of known patterns, such as stripes or grids, onto the surgical site, using a projector of the surgical data fusion system 110 or surgical imaging system 100. Accordingly, the surgical imaging system 100 or surgical data fusion system 110 may comprise a structured light emitter configured to emit structured light towards the surgical site. A camera, such as the surgical imaging device 120 or an overview camera, observes how this pattern deforms as it lands on surfaces. The surgical data fusion system 110 may use triangulation to calculate the depth of each point based on the amount of distortion. The surgical data fusion system 110 may be configured to measure the displacement of the projected pattern to determine the distance of different points of the surgical site vis-à-vis the camera. This knowledge may be used to generate the three-dimensional depth map of the sample.

Operation b) (how and where the one or more intraoperative measurements are to be visualized relative to the at least one planar visual representation of at least one slice of the three-dimensional anatomical information) depends on the slice or slices of the three-dimensional anatomical information and on the extent of the respective visual representation of the intraoperative measurement(s). For example, the surgical data fusion system 110 may be configured to determine a spatial relationship between the at least one slice of the three-dimensional anatomical information and the one or more intraoperative measurements, e.g., based on the determined spatial relationship of the one or more intraoperative measurements to the three-dimensional anatomical information as a whole. Thus, the position information may comprise information on a spatial relationship (e.g., including relative position and/or relative rotational offset) between the at least one slice of the three-dimensional anatomical information and the one or more intraoperative measurements. Additionally, the surgical data fusion system 110 may be configured to determine an extent (e.g., in two dimensions in the same plane(s) as the at least one slice, or in three dimensions relative to the plane(s) of the at least one slice) of the visual representation of the one or more intraoperative measurements (which is inherent to the one or more intraoperative measurement), to determine how the visual representation of the one or more intraoperative measurements intersects with the planar visual representation of the at least one slice. Thus, the position information may comprise information on how the visual representation of the one or more intraoperative measurements intersects with the planar visual representation of the at least one slice.

In some examples, the surgical data fusion system 110 acts as a surgical visualization system and provides the surgical visualization. In other words, the surgical data fusion system 110 may be configured to generate a surgical visualization 20 of the surgical site 10. This surgical visualization is based on the position information, considering the planar visual representation(s) of the at least one slice and the visual representation of the one or more intraoperative measurements. In particular, the surgical visualization may comprise the at least one planar visual representation 30 of the at least one slice of the three-dimensional anatomical information, overlaid with the visual representation 40 of the one or more intraoperative measurements. For example, the surgical data fusion system 110 may be configured to place the overlay on the at least one planar visual representation based on the position information.

A major application of the proposed concept is the combined visualization of live image data of the surgical imaging device and of the planar visual representation of the respective slice(s) of the three-dimensional anatomical information. Accordingly, the visual representation of the one or more intraoperative measurements may be based on the image data of the surgical imaging device, showing the surgical site. Examples of such a visualization are shown in Figs. 1b, 2 and 3b, for example. To improve the fidelity of such a visualization, the aforementioned three-dimensional depth map may be used to generate the visual representation of the image data. For example, the surgical data fusion system may be configured to use image projection to map the image data onto a three-dimensional depth map of the surgical site, with the visual representation of the one or more intraoperative measurements being based on the image data mapped onto the three-dimensional depth map of the surgical site. In general, image projection is the process of mapping points from one space into another space. In many cases, image projection is used to project a three-dimensional object onto a two-dimensional image plane, simulating how a two-dimensional camera captures a three-dimensional scene. In the proposed concept, a 2D to 3D (from the image data onto the depth map) to 2D (from the depth map to a 2D version of the visual representation of the image data to be included in the surgical visualization) projection may be used. For example, mathematical operations may be used to project the image information contained in the image data onto the depth map, e.g., based on the disparity map being used to generate the three-dimensional depth map, or using feature mapping. Then, mathematical operations may be used to project the image information that is contained in the three-dimensional depth map of the surgical site into a corresponding two-dimensional visual representation of the image data, which is based on the position information, as the position information indicates the angle of view on the surgical site that is used for the surgical visualization. These mathematical operations are based on the perspective of the respective two-dimensional image relative to the three-dimensional object. In particular, the pixels of the image data may first be projected onto the three-dimensional depth map, and then re-projected from the three-dimensional depth map onto the image plane(s) used for the surgical visualization. The image plane(s) used for the surgical visualization, on the other hand, are based on the planes of the planar visual representation(s) of the three-dimensional depth map.

The same technique may be used for other types of intraoperative measurements. First, the respective measurements may be mapped onto the three-dimensional depth map of the surgical site and then mapped into the image plane(s) used for surgical visualization. Accordingly, the surgical data fusion system 110 may be configured to generate the surgical visualization based on the three-dimensional depth map of the surgical site.

In general, the fusion of the visual representation of the one or more intraoperative measurements and the planar visual representation of the at least one slice may grant precedence to the visual representation of the one or more intraoperative measurements, as the one or more intraoperative measurements may be real-time measurements and thus reflect the current state of the surgical site. Thus, the shape of the surgical site, as represented in the one or more intraoperative measurements, may be used to replace the corresponding portions of the planar visual representation(s) of the slice(s) of the three-dimensional anatomical information. For example, the surgical data fusion system 110 may be configured to generate the visual representation of the one or more intraoperative measurements based on a shape of the surgical site at the position of the at least one slice of the three-dimensional anatomical information. For example, a portion of the planar visual representation of the respective slice may be replaced by (e.g., overlaid with) the visual representation of the one or more intraoperative measurements according to the shape of the surgical site at the position of the at least one slice of the three-dimensional anatomical information.

As outlined above, the one or more intraoperative measurements may be more up to date than the pre- or intraoperative scans of the patient included in the three-dimensional anatomical information. In particular, the anatomy of the patient may have changed to access the surgical site during surgery, e.g., as part of the skull has been removed or as tissue has been moved aside. As these changes are represented in the one or more intraoperative measurements, they may also be introduced into the planar visual representation(s) of the respective slice(s). In other words, the surgical data fusion system may be configured to determine, based on the three-dimensional anatomical information and the three-dimensional depth map of the surgical site, a portion of the surgical site that is removed compared to the pre-operative scan. Again, this can be done by comparing the anatomical features included in the three-dimensional anatomical information with the anatomical features represented in the one or more intraoperative measurements (e.g., in the three-dimensional depth map generated based on the one or more intraoperative measurements). The surgical data fusion system 110 may be configured to generate the at least one planar visual representation of the at least one slice of the three-dimensional anatomical information without the portion of the surgical site that may be removed. Similarly, changes that are not attributable to the removal of a portion of the surgical site can be tracked as well. In particular, the surgical data fusion system may be configured to determine, based on the three-dimensional anatomical information and the three-dimensional depth map of the surgical site, a shift or deformation of at least a portion of the surgical site, and to adjust the at least one planar visual representation of the at least one slice of the three-dimensional anatomical information based on the shift or deformation of least the portion of the surgical site.

The surgical data fusion system 110 may be configured to provide the surgical visualization for a display device, such as the ocular displays 130a or the auxiliary display 130b. For example, the surgical data fusion system 110 may be configured to output a display signal comprising the surgical visualization for the display device. For example, the display signal may be a signal for driving (e.g., controlling) the respective display device. For example, the display signal may comprise video data and/or control instructions for driving the display. For example, the display signal may be provided via one of the one or more interfaces 112 of the system. Accordingly, the surgical data fusion system 110 may comprise a video interface 112 that is suitable for providing the display signal to the display devices 130a, 130b of the microscope system 100.

In the proposed surgical imaging system, optical imaging sensor(s) of the surgical imaging device 120 may be used to provide the aforementioned image data. Accordingly, the optical imaging sensor(s), which may be part of the imaging device 120 (e.g., of the microscope) may be configured to generate the image data. For example, the optical imaging sensor(s) of the imaging device 120 may comprise or be an APS (Active Pixel Sensor)-based imaging sensor or a CCD (Charge-Coupled-Device)-based imaging sensor. For example, in APS-based imaging sensors, light is recorded at each pixel using a photodetector and an active amplifier of the pixel. APS-based imaging sensors are often based on CMOS (Complementary Metal-Oxide-Semiconductor) or S-CMOS (Scientific CMOS) technology. In CCD-based imaging sensors, incoming photons are converted into electron charges at a semiconductor-oxide interface, which are subsequently moved between capacitive bins in the imaging sensor by circuitry of the imaging sensor to perform the imaging.

The one or more interfaces 112 of the system 110 may correspond to one or more inputs and/or outputs for receiving and/or transmitting information, which may be in digital (bit) values according to a specified code, within a module, between modules or between modules of different entities. For example, the one or more interfaces 112 may comprise interface circuitry configured to receive and/or transmit information. The one or more processors 114 of the system 110 may be implemented using one or more processing units, one or more processing devices, any means for processing, such as a processor, a computer or a programmable hardware component being operable with accordingly adapted software. In other words, the described function of the one or more processors 114 may as well be implemented in software, which is then executed on one or more programmable hardware components. Such hardware components may comprise a general-purpose processor, a Digital Signal Processor (DSP), a micro-controller, etc. The one or more storage devices 116 of the system 110 may comprise at least one element of the group of a computer readable storage medium, such as a magnetic or optical storage medium, e.g., a hard disk drive, a flash memory, Floppy-Disk, Random Access Memory (RAM), Programmable Read Only Memory (PROM), Erasable Programmable Read Only Memory (EPROM), an Electronically Erasable Programmable Read Only Memory (EEPROM), or a network storage.

More details and aspects of the surgical data fusion system 110 and the surgical imaging system 100 are mentioned in connection with the proposed concept or one or more examples described above or below (e.g., Figs. 2 to 5). The surgical data fusion system 110 and the surgical imaging system 100 may comprise one or more additional optional features corresponding to one or more aspects of the proposed concept or one or more examples described above or below.

Various examples of the present disclosure relate to the integration of radiological cross-section visualization with real-time surgical microscopy (radiology-like 3D visualization) or other intraoperative measurements. The proposed concept details a new visualization method that combines preoperative 3D imaging data with real-time optical imaging obtained from the surgical microscope (or other measurements). This data is presented in the form of radiological cross-sectional planes, which may include axial, coronal, and sagittal views. The system may capture the 3D surface of the surgical cavity intraoperatively, utilizing methods such as stereo optical imaging, structured light projection (SLP), or time-of-flight (TOF) sensors. This allows the system to align and integrate the real-time optical data or other measurements with the preoperative 3D data. The resulting hybrid visualization provides surgeons with an augmented reality view that incorporates the familiar and precise radiological cross-sections, enabling intuitive comprehension and measurement of 3D spatial relationships during surgery.

Fig. 2 shows an example of radiological cross-sections augmented with real-time optical imaging. This figure illustrates how radiological cross-sectional views 30a-30c - axial 30c, coronal 30a, and sagittal 30b - are integrated with real-time optical imaging data 40a, 40b, 40c. The different planes chosen (coronal 200a, sagittal 200b and axial 200c) are shown in the respective bottom right. In the example shown in Fig. 2, the system aligns the live optical data with preoperative 3D scans, maintaining the familiar radiological perspective while simultaneously providing updated and accurate views during surgery. On the left, a standard visualization of medical 3D scan data is shown, on the right, the proposed hybrid optical-3D medical imaging visualization.

The proposed concept provides a mechanism for enhancing intraoperative visualization by integrating radiological cross-sectional imaging, e.g., with real-time optical imaging obtained from a surgical microscope or other intraoperative measurements. This approach leverages the strengths of both visualization methods, providing surgeons with detailed spatial information in a familiar format, while simultaneously maintaining a realistic view of the surgical field.

The system is based on one or more key components. For example, a surgical imaging device, such as a surgical microscope, with 3D surface capture capability may be utilized. This surgical imaging device may be equipped with technologies such as stereo optical imaging, structured light projection (SLP), time-of-flight (ToF) sensors, or other 3D scanning technologies, enabling it to capture the real-time 3D surface of the surgical cavity intraoperatively (e.g., as part of the one or more intraoperative measurements).

Pre- or intraoperative operative 3D imaging data (e.g., the three-dimensional anatomical information), obtained from modalities like MRI (Magnetic Resonance Imaging) or CT (Computed Tomography) scans, may provide detailed anatomical and pathological structures relevant to the surgery. An Image-Guided Surgery (IGS) system may be used for the spatial alignment (registration) of the intraoperative 3D surface data with the preoperative 3D imaging data. This system may also provide tracking of surgical instruments and real-time updates of the combined data.

Finally, a visualization module, such as the surgical data fusion system 110, may be used to process and combine the aligned data to generate radiological cross-sectional views in axial, coronal, and sagittal planes and integrate these views with the real-time optical imaging to create an augmented reality display.

The workflow and implementation of the system may proceed as follows. The surgical imaging device may be used to continuously capture the 3D surface of the surgical cavity during the procedure, utilizing methods such as stereo imaging to generate a real-time surface map. The IGS system may be used to align the intraoperative 3D surface data with the preoperative 3D imaging data, achieving spatial alignment through the use of fiducial markers, anatomical landmarks, or surface matching algorithms. The aligned 3D data may be processed to extract cross-sectional images in the axial, coronal, and sagittal planes, incorporating both the preoperative data and the intraoperative optical surface data. These cross-sectional images may be overlaid onto the live optical feed from the surgical microscope, allowing surgeons to view the combined data in familiar radiological formats directly within the surgical field.

In some examples, interactive features may be included, allowing surgeons to adjust the position of the cross-sectional planes in real-time to focus on areas of interest, utilize measurement tools to quantify distances and spatial relationships, and display all three planes simultaneously or toggle between them.

Figs. 3a and 3b illustrate radiological cross-sectional visualization overlaid on real-time optical imaging. Fig. 3a illustrates the planes 200a, 200b, 200c defining radiological cross-sectional views, while Fig. 3b shows a combined view comprising radiological cross-sectional views and a live optical feed. Sliders 300a, 300b, 300c may be used to shift the coronal plane 200a (slider 300a), the sagittal plane 200b (slider 300b), and/or the axial plane 200c (slider 300c). Figs. 3a and 3b demonstrate the overlay of radiological cross-sectional views 30 (axial, coronal, sagittal) of a preoperatively acquired 3D scan onto the live optical feed 40 (optical image data) from the stereo surgical microscope (or vice versa). The combined view provides surgeons with both a realistic optical view of the surgical field and precise spatial information derived from the preoperative 3D data. The system's interactive features allow surgeons to explore different planes and focus on areas of interest during surgery.

The proposed mechanism provides a familiar visualization format, presenting surgeons with radiological cross-sectional views they are accustomed to interpreting, thereby enhancing understanding. It enhances 3D spatial comprehension by combining real-time optical data with preoperative imaging, offering precise spatial relationships between structures. Furthermore, it improves surgical planning and execution, allowing for accurate navigation and manipulation of instruments relative to critical anatomical features. Moreover, it reduces cognitive load by eliminating the need for mental mapping between separate displays, streamlining the workflow. Finally, it offers versatility, as it can be integrated into existing IGS systems and surgical microscopes with 3D imaging capabilities.

Several alternative implementations are possible. Different 3D surface capture methods can be employed; while stereo optical imaging is one option, structured light projection (SLP) or time-of-flight (TOF) sensors can also be used for capturing the 3D surface. Advanced visualization techniques, such as incorporating volume rendering or multi-planar reconstructions, can enhance the visualization. User interface customization can be provided, allowing surgeons to customize display settings such as opacity levels, color schemes, and plane orientations. Finally, integration with other modalities, including functional imaging data (e.g., fMRI, DTI), can provide additional information about tissue functionality.

The proposed concept integrates radiological cross-sectional visualization with real-time optical imaging in surgical microscopes. It offers augmented reality views combining axial, coronal, and/or sagittal planes with live optical imaging. It provides radiology-inspired visualization, enhanced 3D spatial understanding, and/or hybrid optical and radiological imaging during surgery.

More details and aspects of the integration of radiological cross-section visualization with real-time surgical microscopy are mentioned in connection with the proposed concept or one or more examples described above or below (e.g., Figs. 1a to 1c, 4 to 5). The integration of radiological cross-section visualization with real-time surgical microscopy may comprise one or more additional optional features corresponding to one or more aspects of the proposed concept or one or more examples described above or below.

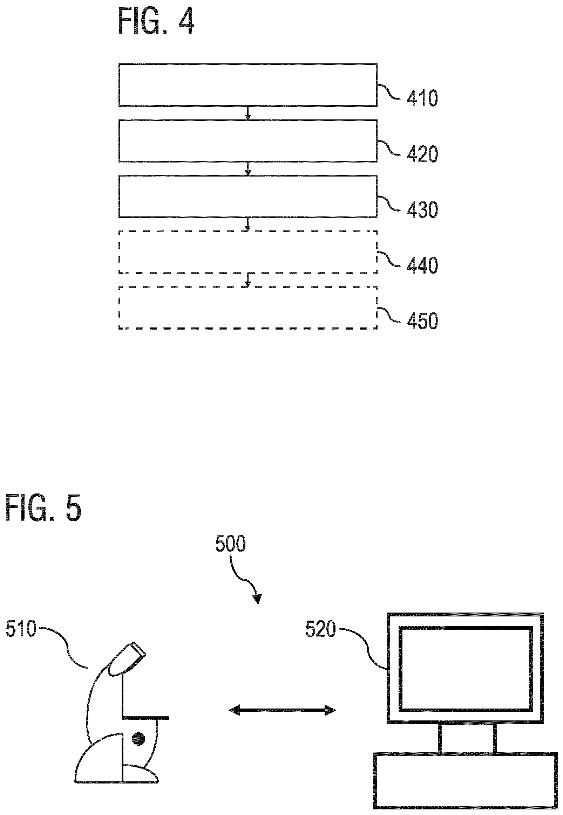

Fig. 4 shows a flow chart of an example of a surgical data fusion method. The method comprises obtaining 410 three-dimensional anatomical information of a patient, the three-dimensional anatomical information being based on a pre-operative or intraoperative scan of a patient. The method comprises obtaining 420 one or more intraoperative measurements of the patient. The method comprises determining 430 position information for a visual representation of the one or more intraoperative measurements relative to the three-dimensional anatomical information. The position information indicates a position of the visual representation 40 of the one or more intraoperative measurements relative to at least one planar visual representation 30 of at least one slice of the three-dimensional anatomical information. Optionally, the method may further comprise generating 440 a surgical visualization 20 of a surgical site and providing 450 the surgical visualization for a display device. For example, the method may be performed by the surgical data fusion system 110 of Figs. 1a and 1b. Features introduced in connection with the surgical data fusion system 110 of Figs. 1a and 1b, or with the example provided in connection with Figs. 2 and 3a to 3b, may likewise be included in the corresponding surgical data fusion method of Fig. 4.

More details and aspects of the surgical data fusion method are mentioned in connection with the proposed concept or one or more examples described above or below (e.g., Figs. 1a to 3b, 5). The method may comprise one or more additional optional features corresponding to one or more aspects of the proposed concept or one or more examples described above or below.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some embodiments relate to an imaging device comprising a system as described in connection with one or more of the Figs. 1a to 4. Alternatively, an imaging device, such as a microscope, exoscope or endoscope, may be part of or connected to a system as described in connection with one or more of the Figs. 1a to 4. Fig. 5 shows a schematic illustration of a system 500 configured to perform a method described herein. The system 500 comprises an imaging device 510 and a computer system 520. The imaging device 510 is configured to take images and is connected to the computer system 520. The computer system 520 is configured to execute at least a part of a method described herein. The computer system 520 may be configured to execute a machine learning algorithm. The computer system 520 and imaging device 510 may be separate entities but can also be integrated together in one common housing. The computer system 520 may be part of a central processing system of the imaging device 510 and/or the computer system 520 may be part of a subcomponent of the imaging device 510, such as a sensor, an actor, a camera, or an illumination unit, etc. of the imaging device 510.

The computer system 520 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 520 may comprise any circuit or combination of circuits. In one embodiment, the computer system 520 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a imaging device or a imaging device component (e.g. camera) or any other type of processor or processing circuit. Other types of circuits that may be included in the computer system 520 may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system 520 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 520 may also include a display device, one or more speakers, and a keyboard and/or controller, which can include a mouse, trackball, touch screen, voice-recognition device, or any other device that permits a system user to input information into and receive information from the computer system 520.

Some or all of the method steps may be executed by (or using) a hardware apparatus, like for example, a processor, a microprocessor, a programmable computer, or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine-readable carrier.

Other embodiments comprise the computer program for performing one of the methods described herein, stored on a machine-readable carrier.

In other words, an embodiment of the present invention is, therefore, a computer program having a program code for performing one of the methods described herein, when the computer program runs on a computer.

A further embodiment of the present invention is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device, or the like. The apparatus or system may, for example, comprise a file server for transferring the computer program to the receiver.

In some embodiments, a programmable logic device (for example, a field programmable gate array) may be used to perform some or all the functionalities of the methods described herein. In some embodiments, a field programmable gate array may cooperate with a microprocessor to perform one of the methods described herein. Generally, the methods are preferably performed by any hardware apparatus.

### List of reference Signs

- 10:: Surgical site
- 20:: Visualization
- 30, 30a, 30b, 30c:: Planar visual representation of a slice of 3D anatomical information
- 40, 40a, 40b, 40c:: Visual representation of one or more intraoperative measurements
- 50:: IGS system
- 100:: Surgical imaging system
- 110:: Surgical data fusion system
- 112:: One or more interfaces
- 114:: One or more processors
- 116:: One or more storage devices
- 120:: Imaging device
- 130a:: Ocular displays
- 130b:: Auxiliary display
- 200a:: Coronal plane
- 200b:: Sagittal plane
- 200c:: Axial plane
- 300a:: Slider for changing coronal plane
- 300b:: Slider for changing sagittal plane
- 300c:: Slider for changing axial plane
- 410:: Obtaining three-dimensional anatomical information
- 420:: Obtaining one or more intraoperative measurements
- 430:: Determining position information
- 440:: Generating a surgical visualization
- 450:: Providing the surgical visualization
- 500:: System
- 510:: Imaging device
- 520:: Computer system

## Claims

1. A surgical data fusion system (110), the surgical data fusion system comprising one or more processors and one or more storage devices, wherein the surgical data fusion system is configured to:
obtain three-dimensional anatomical information of a patient, the three-dimensional anatomical information being based on a pre-operative or intraoperative scan of a patient;
obtain one or more intraoperative measurements of the patient; and
determine position information for a visual representation (40) of the one or more intraoperative measurements relative to the three-dimensional anatomical information, wherein the position information indicates a position of the visual representation (40) of the one or more intraoperative measurements relative to at least one planar visual representation (30) of at least one slice of the three-dimensional anatomical information.

2. The surgical data fusion system according to claim 1, wherein the at least one planar visual representation mimics at least one cross-sectional radiological image.

3. The surgical data fusion system according to one of the claims 1 or 2, wherein the at least one planar visual representation represents at least one of an axial plane, a coronal plane, or a sagittal plane of the three-dimensional anatomical information.

4. The surgical data fusion system according to claim 1, wherein the surgical data fusion system is configured to generate a surgical visualization (20) of a surgical site (10), the surgical visualization comprising the at least one planar visual representation (30) of the at least one slice of the three-dimensional anatomical information, overlaid with the visual representation (40) of the one or more intraoperative measurements, with the overlay being placed on the at least one planar visual representation based on the position information, and to provide the surgical visualization for a display device (130a, 130b).

5. The surgical data fusion system according to claim 4, wherein the surgical data fusion system is configured to obtain a user input of a user of the surgical data fusion system, and to select the at least one slice of the three-dimensional anatomical information for the surgical visualization based on the user input.

6. The surgical data fusion system according to one of the claims 4 or 5, wherein the one or more intraoperative measurements of the patient comprise image data of a surgical imaging device, the image data showing the surgical site, with the visual representation of the one or more intraoperative measurements being based on the image data.

7. The surgical data fusion system according one of the claims 4 to 6, wherein the surgical data fusion system is configured to determine a three-dimensional depth map of the surgical site, and generate the surgical visualization based on the three-dimensional depth map of the surgical site.

8. The surgical data fusion system according to claim 7, wherein the surgical data fusion system is configured to generate the visual representation of the one or more intraoperative measurements based on a shape of the surgical site at the position of the at least one slice of the three-dimensional anatomical information.

9. The surgical data fusion system according to one of the claims 7 or 8, wherein the surgical data fusion system is configured to determine, based on the three-dimensional anatomical information and the three-dimensional depth map of the surgical site, a portion of the surgical site that is removed compared to the pre-operative scan, and to generate the at least one planar visual representation of the at least one slice of the three-dimensional anatomical information without the portion of the surgical site that is removed,
and/or wherein the surgical data fusion system is configured to determine, based on the three-dimensional anatomical information and the three-dimensional depth map of the surgical site, a shift or deformation of at least a portion of the surgical site, and to adjust the at least one planar visual representation of the at least one slice of the three-dimensional anatomical information based on the shift or deformation of least the portion of the surgical site.

10. The surgical data fusion system according to one of the claims 7 to 9, wherein the surgical data fusion system is configured to determine the three-dimensional depth map of the surgical site using stereo disparity analysis based on image frames contained in image data of a surgical imaging device showing the surgical site from different angles of view,
or wherein the surgical data fusion system is configured to determine the three-dimensional depth map using at least one of sensor data of a time-of-flight sensor or image data showing a structured light projection.

11. The surgical data fusion system according to one of the claims 1 to 10, wherein the surgical data fusion system is configured to obtain the three-dimensional anatomical information of an image-guided surgery system (50), and to determine the position information based on a registration functionality of the image-guided surgery system.

12. The surgical data fusion system according to one of the claims 1 to 11, wherein the one or more intraoperative measurements of the patient comprise functional imaging data representing a brain activity of the patient and the three-dimensional anatomical information comprises three-dimensional anatomical information of the brain of the patient.

13. A surgical imaging system (100) comprising a surgical imaging device (120) and the surgical data fusion system (110) according to one of the claims 1 to 12.

14. A surgical data fusion method comprising:
obtaining (210) three-dimensional anatomical information of a patient, the three-dimensional anatomical information being based on a pre-operative or intraoperative scan of a patient;
obtaining (220) one or more intraoperative measurements of the patient;
determining (230) position information for a visual representation of the one or more intraoperative measurements relative to the three-dimensional anatomical information, wherein the position information indicates a position of the visual representation (40) of the one or more intraoperative measurements relative to at least one planar visual representation (30) of at least one slice of the three-dimensional anatomical information.

15. A computer program having a program code for performing a method according to claim 14 when the program is executed on a processor.
